(19) Europäisches Patentamt
European Patent Office
Office européen des brevets

(11) Publication number: **0 339 681 B1**

(12) **EUROPEAN PATENT SPECIFICATION**

(45) Date of publication of patent specification: **31.03.93**

(51) Int. Cl.⁵: **C07D 487/04**, A61K 31/40, //(C07D487/04,209:00,209:00)

(21) Application number: **89107799.2**

(22) Date of filing: **28.04.89**

(54) **Antitumor antibiotic substance.**

(30) Priority: **28.04.88 JP 103782/88**

(43) Date of publication of application: **02.11.89 Bulletin 89/44**

(45) Publication of the grant of the patent: **31.03.93 Bulletin 93/13**

(84) Designated Contracting States: **DE FR GB**

(56) References cited:
**EP-A- 0 154 445**
**WO-A-88/04659**

**JOURNAL OF THE AMERICAN CHEMICAL SOCIETY, vol. 109, 1987; R.C.KELLY et al.: "Coupling of cyclopropa pyrroloindole (CPI) derivatives. The preparation of CC-1065, ENT-CC-1065, and analogues", pp. 6837-6838**

**THE JOURNAL OF ANTIBIOTICS, vol. 41, no. 10, 1988, pp. 1515-1519**

(73) Proprietor: **MEIJI SEIKA KAISHA LTD.
4-16 Kyobashi 2-chome
Chuo-ku Tokyo(JP)**

(72) Inventor: **Koyama, Masao c/o Meiji Seika Kaisha, Ltd.
Pharmaceutical Research Lab. 760, Morooka-cho
Kohoku-ku Yokohama-shi Kanagawa(JP)**
Inventor: **Ohba, Kazunori c/o Meiji Seika Kaisha, Ltd.
Pharmaceutical Research Lab. 760, Morooka-cho
Kohoku-ku Yokohama-shi Kanagawa(JP)**
Inventor: **Nakazawa, Tadashi c/o Meiji Seika Kaisha, Ltd.
Pharmaceutical Research Lab. 760, Morooka-cho
Kohoku-ku Yokohama-shi Kanagawa(JP)**
Inventor: **Yamamoto, Haruo c/o Meiji Seika Kaisha, Ltd.
Pharmaceutical Research Lab. 760, Morooka-cho
Kohoku-ku Yokohama-shi Kanagawa(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 339 681 B1

Inventor: **Sezaki, Masaji c/o Meiji Seika Kaisha, Ltd.**
**Pharmaceutical Research Lab. 760, Morooka-cho**
**Kohoku-ku Yokohama-shi Kanagawa(JP)**
Inventor: **Kondo, Shinichi c/o Meiji Seika Kaisha, Ltd.**
**Pharmaceutical Research Lab. 760, Morooka-cho**
**Kohoku-ku Yokohama-shi Kanagawa(JP)**

(74) Representative: **Wilhelms, Rolf E., Dr. et al**
**WILHELMS, KILIAN & PARTNER Patentanwälte Eduard-Schmid-Strasse 2**
**W-8000 München 90 (DE)**

## Description

### FIELD OF THE INVENTION

This invention relates to an antibiotic substance having an antitumor activity.

### BACKGROUND OF THE INVENTION

Three antibiotic substances are isolated from a culture medium of a strain SF2582 belonging to the genus Streptomyces which has been deposited at Agency of Fermentation Research Institute of Japan under the accession No. FERM BP-2087 in accordance with the Budapest Treaty. Each of the substances (hereinafter referred to as SF2582A, B and C) show a remarkable cytotoxic effect on various tumor cells, and are proved to have a therapeutic effect on infection in an experiment using mice (as described in K. Ohba et al., The Journal of Antibiotics, 41 (10), 1515-1519 (1988)).

The substances SF2582A, B and C have structures represented by the following formulae (III), (IV) and (V) respectively.

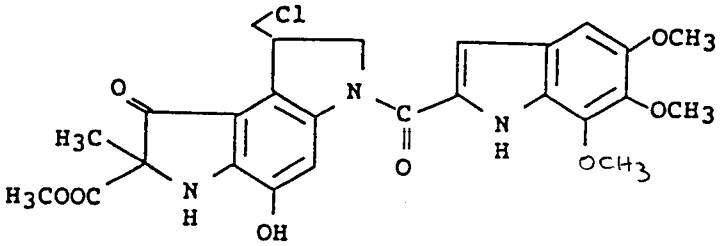

(III)

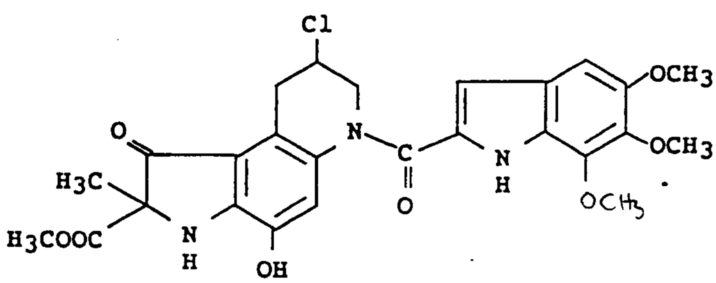

(IV)

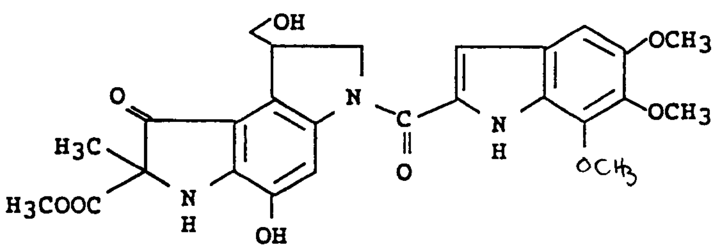

(V)

Although the substance SF2582C is closely similar to the SF2582A and SF2582B in structure, the antitumor activity of the former is much lower than those of the latter ones. Thus, the substance SF2582C scarcely shows any remarkable cytotoxic effect on test tumor cells at a low concentration. In order to effectively utilize the substance SF2582, it is required, therefore, not only to produce the same by a microbial fermentation process but also to chemically modify the substance SF2582C to obtain derivatives thereof which have the enhanced antitumor activity and other physiological activities.

## SUMMARY OF THE INVENTION

We assumed that the difference in the antitumor activities of the substances SF2582A and SF2582B and the substance SF2582C might relate to the presence of a chlorine atom in a molecule. Based on this assumption, we have attempted to introduce an active group comparable to a chlorine atom into the SF2582C molecule through chemical modification to thereby develop a novel active compound. Thus we have examined various chemical reactivities of the starting material, namely, the substance SF2582C, synthesized a number of derivatives of the same and evaluated the antitumor activities of the derivatives thus obtained. As a result, we have found that a sulfate or an alkyl- or arylsulfonate of the SF2582C substance shows an intense antitumor activity.

## DETAILED DESCRIPTION OF THE INVENTION

Novel SF2582C derivatives according to the present invention which are obtained by chemical modification of the substance SF2582C represented by the following formula (I):

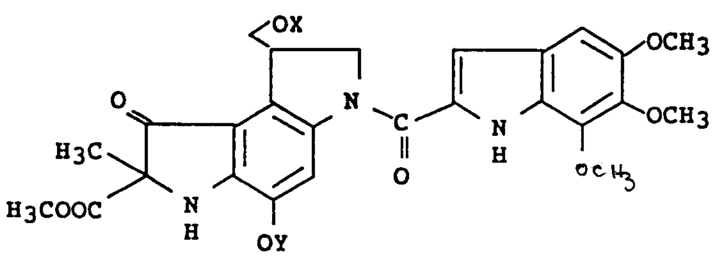

(I)

wherein X and Y represents each an $RSO_2$-group, wherein R represents a p-tolyl, methyl or hydroxyl group, or a hydrogen atom, provided that X and Y do not represent hydrogen atoms at the same time.

A preferable example of $RSO_2$-group represented by X and Y is a methanesulfonyl group.

The starting material of the compound of the formula (I), the substance SF2582C, can be isolated from the culture of strain SF2582 (FERM BP-2087).

The strain is cultured in a medium containing nutrients utilizable by general microorganisms. Known nutrient sources conventionally employed for cultivation of actinomycetes can be used. Specific examples of the nutrient sources are carbon sources, e.g., glucose, maltose syrup, dextrin, starch, molasses, and animal and vegetable oils; and nitrogen sources, e.g., soybean flour, wheat germ, corn steep liquor, cotton seedmeal, meat extract, peptone, yeast extract, ammonium sulfate, sodium nitrate, and urea. If desired, the medium can contain inorganic salts capable of providing a sodium ion, a potassium ion, a calcium ion, a magnesium ion, a cobalt ion, a chlorine ion, a phosphate ion, a sulfate ion or other ions. Further, organic or inorganic substances effective to assist cell growth and to accelerate production of SF2582C can be added to the medium appropriately.

The cultivation can be suitably effected under aerobic conditions, and most suitably by submerged aeration culture. A suitable cultivation temperature is from 26 to 30° C and, in most cases, around 28° C. The production of SF2582C varies depending on the medium or cultivation conditions. Usually, the accumulation reaches the maximum in 2 to 7 days by shake culture or tank culture when the accumulated amount in the culture reaches the maximum, cultivation is ceased, and the desired substance is isolated from the culture and purified.

Isolation of the substance SF2582C from the culture can be carried out by usual means taking advantage of the properties of the desired substance, such as filtration, solvent extraction, adsorption or partition column chromatography, gel filtration, dialysis, precipitation, or the like technique, either alone or in combination thereof. For example, the substance SF2582C can be extracted with acetone-water or methanol-water from the fungus body. The aqueous extract is then extracted with a water-immiscible organic solvent, e.g., butanol and ethyl acetate, thereby passing the active components into the organic layer. The substance SF2582C can further be purified by column chromatography on an adsorbent, e.g., silica gel ("Wako Gel C-200" made by Wako Pure Chemical Inds., Ltd.) and alumina, Sephadex LH-20 (made by Pharmacia Co.), etc.

The compound of the formula (I) provided by the present invention may be produced by reacting the substance SF2582C with an esterifying agent such as sulfonic acid anhydride, sulfonyl halide, sulfuric acid

anhydride/pyridine complex or chlorosulfonic acid in an inert solvent in the presence of a base to thereby esterify a hydroxyl group. Sulfonic acid anhydride and sulfonyl halide are preferably used as an esterifying agent. Examples of the inert solvent include benzene, toluene, tetrahydrofuran, dioxane, pyridine and N,N-dimethylformamide. Preferable inert solvents are tetrahydrofuran, pyridine and N,N-dimethylformamide. Examples of the base include alkylamines such as triethylamine and pyridine bases, preferably pyridine bases. The esterification would readily proceed at room temperature. In the case that a monoester is to be produced, however, it is preferable to carry out the above reaction at a temperature ranging from 20 to 25 ° C for 1 to 4 hours with the use of an esterifying agent in a restricted amount, preferably 1 to 1.2 time by mol as much as the starting material, since the substance SF2582C carries two hydroxyl groups per molecule, as shown in the formula (V).

When the formula (I) is a monoester of the substance SF2582C, X represents a hydrogen atom and Y represents an $RSO_2$-group.

To illustrate the usefulness of the derivatives of the present invention, the following Examples and Test Examples will be given.

EXAMPLE 1

Production of SF2582C monomethanesulfonate (Compound 2) and SF2582C dimethanesulfonate (Compound 1)

40 mg of the substance SF2582C as obtained in Reference Example below was dissolved in 5 ml of anhydrous N,N-dimethylformamide and 0.1 ml of triethylamine and 50 mg of methanesulfonic anhydride were added thereto. The obtained mixture was allowed to react at 25 ° C for ten minutes. The reaction mixture was extracted with 20 ml of benzene and 20 ml of water and the benzene phase was separated, washed with water and dried by adding anhydrous sodium sulfate thereto. After filtering off the sodium sulfate, the benzene solution was concentrated under reduced pressure. Thus, a yellow solid was obtained as the residue. This product was further purified by silica gel chromatography (solvent: benzene/ethyl acetate (1 : 1)) to thereby obtain the following three fractions.

Fraction 1: SF2582C dimethanesulfonate (Compound 1)
yield: 41 mg.
$^1$H NMR ($CDCl_3$, ppm): 9.36 (s), 8.60 (s), 6.95 (d), 6.86 (s), 5.76 (s), 4.77 (dd), 4.65 (d), 4.48 (dd), 4.21 (m), 4.07 (s), 3.94 (s), 3.91 (s), 3.78 (s), 3.32 (s), 3.03 (s) and 1.70 (s).
FD mass: 682 $(M+1)^+$.

Fraction 2: a mixture of Compounds 1 and 2
yield: 6.3 mg.

Fraction 3: SF2582C monomethanesulfonate (Compound 2)
yield: 3.7 mg.
$^1$H NMR ($CDCl_3$, ppm): 9.73 (2), 8.60 (s), 6.95 (d), 6.83 (s), 5.73 (s), 4.59 (d), 4.07 (s), 4.01 (m), 3.93 (s), 3.91 (m), 3.90 (s), 3.87 (m), 3.77 (s), 3.31 (s) and 1.69 (s).
FD mass: 603 $(M)^+$.

EXAMPLE 2

Production of SF2582C monotoluenesulfonate (Compound 3)

10 mg of the substance SF2582C was dissolved in 1.5 ml of anhydrous N,N-dimethylformamide and 0.2 ml of triethylamine and 10 mg of p-toluenesulfonyl chloride were added thereto. The obtained mixture was allowed to react at 25 ° C for 30 minutes. The reaction mixture was extracted with 20 ml of benzene and 20ml of water and the benzene phase was separated, washed with water and dried by adding anhydrous sodium sulfate thereto. Then the benzene solution was concentrated under reduced pressure. Thus a yellow solid was obtained as the residue. This product was further purified by silica gel chromatography (solvent: benzene/ethyl acetate (1 : 1)) to thereby give 12.6 mg of SF2582C mono-p-toluenesulfonate.

$^1$H NMR $CDCl_3$, ppm): 9.31 (s), 8.23 (s), 7.87 (d), 7.38 (d), 6.91 (d), 6.82 (s), 5.65 (s), 4.53 (m), 4.07 (s), 3.98 (s), 3.90 (s), 3.77 (s), 2.47 (s) an 1.64 (s).
FD mass: 679 $(M)^+$.

EXAMPLE 3

Production of SF2582C sulfate (Compound 4)

5 mg of the substance SF2582C was dissolved in 1 ml of anhydrous pyridine and 15 mg of a sulfuric anhydride/pyridine complex was added thereto. The obtained mixture was allowed to react at 25 °C for 18 hours. The reaction mixture was concentrated under reduced pressure and the residue thus obtained was purified by Sephadex LH-20 column chromatography (solvent: methanol/water (1 : 1)). Then a fraction showing an Rf value of 0.03 in silica gel TLC (mfd. by Merck Co., Inc., solvent: chloroform/methanol (9 : 1)) was collected. After removing the methanol under reduced pressure, the residue was lyophilized. Thus, 6 mg of SF2582C sulfate (pyridine salt) was obtained.

SI mass: 606 $(M+1)^+$.

TEST EXAMPLE 1

Cytotoxic effect of Compounds 1 to 4 as obtained in Examples 1 to 3 were evaluated by incubating P-388 mouse leukemia cells at 37 °C for 72 hours in 5% $CO_2$ in the presence of each compound, and calculating a 50% growth inhibition concentration on P-388 cells. The results are shown in Table 1.

Table 1

| Compound No. | $IC_{50}$ (ng/ml) |
|---|---|
| 1 | 60 |
| 2 | 4 |
| 3 | 2500 |
| 4 | 1000 |
| SF2582C (control) | 9400 |

TEST EXAMPLE 2

To evaluate antitumor activity of the test compounds, the following therapeutic test was conducted.

P-388 tumor cells were transplanted into the abdominal cavity of a mouse. A test compound was intraperitoneally administered to the animal once a day for two days. The effect thus observed is expressed in ILS (%).

$$\text{ILS (\%)} = \frac{\text{Average life-elongation (days) of test group}}{\text{Average survival time (days) of control group}} \times 100$$

The results are shown in Table 2.

Table 2

| Compound No. | Dose (mg/kg) | ILS (%) |
|---|---|---|
| 1 | 21 | 54 |
| 1 | 7 | 40 |
| 1 | 2.4 | 40 |
| 2 | 1 | 40 |
| 2 | 0.5 | 40 |
| 2 | 0.25 | 15 |
| Note: Each group had five animals. | | |

Tables 1 and 2 obviously indicate that the compounds of the present invention have each an intense antitumor activity. Thus, these compounds are expected as highly effective antitumor agents.

REFERENCE EXAMPLE

A seed culture medium comprised 1.0% of starch, 1.0% of glucose, 1.0% of wheat germ, 1.0% of Pharmamedia, 0.2% of Staminol (produced by Sapporo Breweries Ltd.), 1.0% of soybean flour, 0.2% of sodium chloride, 0.2% of dipotassium hydrogenphosphate, and 0.05% of magnesium sulfate.

A production culture medium comprised 5.0% of starch maltose syrup, 0.5% of gluten meal, 1.0% of soybean flour, 0.5% of meat extract, 0.2% of Staminol, 0.1% of magnesium sulfate heptahydrate, 0.2% of sodium chloride, and 0.001% of cobalt chloride hexahydrate.

Before sterilization, the seed culture medium and the production culture medium were adjusted to a pH of 7.0 and 6.5, respectively.

A 100 ml-volume Erlenmeyer flask containing 20 ml of the seed medium was sterilized at 120°C for 30 minutes, and the medium was inoculated with 2 to 3 (platinum) loopfuls of a slant agar culture of Streptomyces sp. SF2582 (FERM BP-2087) and shake-cultured at 28°C for 3 days to prepare a first seed culture.

A 500 ml-volume Erlenmeyer flask containing 80 ml of the seed medium was sterilized at 120°C for 30 minutes, and 2.4 ml of the above prepared first seed culture was inoculated thereon, followed by shake-culturing at 28°C for 1 day to obtain a second seed culture.

The cell cake thus obtained was extracted with 350 l of a 60% acetone aqueous solution. The extract containing the active components was concentrated under reduced pressure to obtain a 100 l of an aqueous solution, which was then extracted with 100 l of ethyl acetate. The ethyl acetate extract was concentrated under reduced pressure to obtain 222 g of an oily substance. To the oily substance was added 1 liter of n-hexane, and the hexane-soluble matter was removed. The hexane-insoluble matter was dried to obtain 96 g of an oily substance. The product was passed through a column containing 3 l of silica gel (Wako Gel C-200), washed with 4 l of chloroform, and developed with a mixed solvent of chloroform-methanol (50:1) (10 l) to collect 500 ml fractions. Active fraction Nos. 12 to 16 containing the substance SF2582C were combined and concentrated to precipitate yellowish orange crystals, which were collected by filtration and dried to obtain 920 mg of crude crystals of the substance SF2582C. The resulting crude crystals (920 mg) were recrystallized twice from a mixed solvent of chloroform-methanol (5:1) to obtain 450 mg of the substance SF2582C as orange crystals.

## Claims

**1.** An antitumor compound represented by the following formula (I):

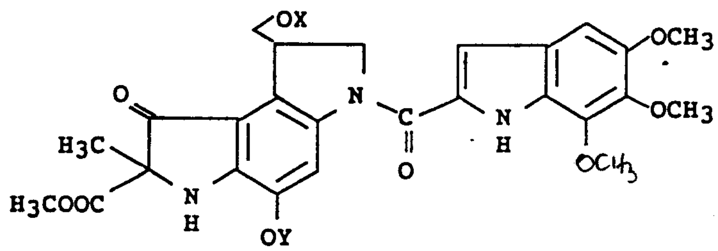

(I)

wherein X and Y represent each an $RSO_2$-group, wherein R represents a tolyl, methyl or hydroxyl group, or a hydrogen atom, provided that X and Y do not represent hydrogen atoms at the same time.

**2.** The antitumor compound according to claim 1, wherein Y represents a methanesulfonyl group.

**3.** The antitumor compound according to claim 2, wherein X represents a hydrogen atom.

**4.** The antitumor compound according to claim 1, wherein X and Y both represent a methanesulfonyl group.

## Patentansprüche

**1.** Antitumorverbindung gemäß der folgenden Formel (I)

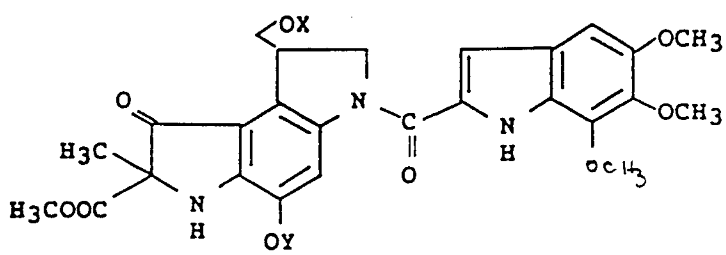

(I)

in der X und Y jeweils eine $RSO_2$-Gruppe sind, in der R eine Tolyl-, Methyl- oder Hydroxylgruppe oder Wasserstoff bedeuten, vorausgesetzt, daß X und Y nicht gleichzeitig für Wasserstoff stehen.

**2.** Antitumorverbindung nach Anspruch 1, wobei Y eine Methansulfonylgruppe ist.

**3.** Antitumorverbindung nach Anspruch 2, wobei X Wasserstoff ist.

**4.** Antitumorverbindung nach Anspruch 1, wobei X und Y beide eine Methansulfonylgruppe sind.

## Revendications

1. Composé antitumoral représenté par la formule suivante (I) :

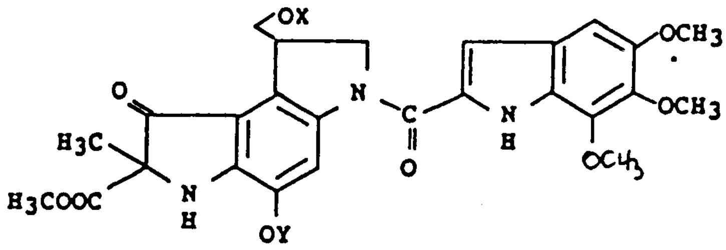

(I)

dans laquelle X et Y représentent chacun un groupe $RSO_2$ dans lequel R représente un groupe tolyle, méthyle ou hydroxyle, ou un atome d'hydrogène, à condition que X et Y ne représentent pas simultanément des atomes d'hydrogène.

2. Composé antitumoral selon la revendication 1, dans lequel Y représente un groupe méthanesulfonyle.

3. Composé antitumoral selon la revendication 2, dans lequel X représente un atome d'hydrogène.

4. Composé antitumoral selon la revendication 1, dans lequel X et Y représentent tous deux un groupe méthanesulfonyle.